(19) Europäisches Patentamt
European Patent Office
Office européen des brevets

(11) EP 0 908 443 A2

(12) EUROPÄISCHE PATENTANMELDUNG

(43) Veröffentlichungstag: **14.04.1999 Patentblatt 1999/15**

(21) Anmeldenummer: **98115767.0**

(22) Anmeldetag: **21.08.1998**

(51) Int. Cl.[6]: **C07C 201/12**, C07C 47/55, C07C 51/62, C07C 253/30, C07C 303/22

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **13.10.1997 DE 19745212**

(71) Anmelder: **Clariant GmbH**
**65929 Frankfurt am Main (DE)**

(72) Erfinder:
- **Schach, Thomas, Dr.**
  **64579 Gernsheim (DE)**
- **Wessel, Thomas, Dr.**
  **60386 Frankfurt (DE)**
- **Gutermuth, Maren**
  **64625 Bensheim (DE)**

Bemerkungen:
Ein Antrag gemäss Regel 88 EPÜ auf Berichtigung von Mängeln in den eingereichten Unterlagen liegt vor. Über diesen Antrag wird im Laufe des Verfahrens vor der Prüfungsabteilung eine Entscheidung getroffen werden (Richtlinien für die Prüfung im EPA, A-V, 3.).

# (54) Verfahren zur Herstellung von fluorierten aromatischen Verbindungen

(57) Verfahren zur Herstellung Von fluorierten Verbindungen

$$Az_xArF_wCl_{(y-w)}R_z \quad (1)$$

worin Az für einen Rest -F, -Cl, -Br, $-NO_2$, -CN, $-CF_3$, $-CCl_3$, -CHO, $-CO(C_nH_{2n+1})$, -COX oder $-SO_2X$ steht, wobei X für F, Cl oder Br steht, x eine ganze Zahl von 1 bis 3 ist, Ar einen Phenyl-Rest, Pyridyl-Rest oder Naphthylrest bedeutet, w eine ganze Zahl von 1 bis y ist, y eine ganze Zahl von 1 bis 5 bedeutet, R für H, einen Alkylrest oder einen Alkoxyrest mit 1 bis 10 Kohlenstoffatomen steht, z eine ganze Zahl von 1 bis 5 ist, ( x+y+z ) die Zahl aller substituierbarer Valenzen am Rest Ar bedeutet, indem man eine Verbindung

$$Az_xArCl_yR_z \quad (2),$$

mit einem Alkalimetallfluorid in Gegenwart einer Komponente a) oder einer Mischung der Komponente a) und wenigstens einer der Komponenten b), c), d) und/oder e) umsetzt, die Komponente a) eine oder mehrere quartäre Ammoniumverbindungen, die eine oder mehrere Reste $-(C_mH_{2m}O)R^5$ enthalten, bedeutet, Komponente b) ein Amidophosphoniumsalz, Komponente c) ein quartäres Ammoniumsalz, Komponente d) ein quartäres Phosphoniumsalz, Komponente e) ein Polyether ist und daß man die Umsetzung bei 50 bis 250°C durchführt, während der Umsetzung die gebildete Verbindung der Formel (1) destillativ abtrennt und die Verbindung der Formel (2), entsprechend wie die Verbindung der Formel (1) abgetrennt wird, der Reaktionsmischung zuführt.

EP 0 908 443 A2

## Beschreibung

Die vorliegende Erfindung betrifft ein neues Verfahren zur Herstellung von fluorierten aromatischen Verbindungen, das die gewünschten Verbindungen durch Halogen-Fluor-Austausch auf schonende Weise zugänglich macht.

Fluorierte aromatische Verbindungen, insbesondere Fluorbenzole bzw. Fluorbenzolderivate, spielen eine bedeutende Rolle als Zwischenprodukte für die Herstellung von Pflanzenschutzmitteln (Herbiziden) und dienen als Synthesebausteine für Pharmazeutika, beispielsweise zur Herstellung von Chinoloncarbonsäuren (Speciality Chemicals, Ed. Brian Pearson, Elsevier 1991, Seiten 15 bis 77) und lassen sich auch als Vorprodukte zur Herstellung von Farbstoffen verwenden.

Der Austausch eines Halogens, vorzugsweise von Chlor oder Brom in aktivierten Chlor- oder Bromaromaten, gegen Fluor stellt eine günstige Methode dar, Fluorsubstituenten in ein aromatisches System einzuführen. Im allgemeinen wird diese Reaktion in Gegenwart von aprotisch dipolaren Lösungsmitteln, die in vergleichsweise hohen Mengen eingesetzt werden, und Alkalimetallfluoriden als Fluoridquelle durchgeführt (US 4,226,811, US 4,069,262).

Die EP-A-0 146 924 betrifft ein Verfahren zur Herstellung einer beta-Fluorpyridinverbindung durch Umsetzen eines alpha- oder gamma-halosubstituierten β-Halopyridins mit KF oder CsF in einem polar aprotischen Lösungsmittel, wobei das beta-Fluorpyridinprodukt im wesentlichen, sobald es gebildet wird, entfernt wird und gegebenenfalls das alpha- oder gamma-halosubstituierte β-Halopyridin zugesetzt wird. Die Umsetzung kann in Anwesenheit eines Phasentransferkatalysators durchgeführt werden, sie kann aber auch - wie die Mehrzahl der Beispiele belegt - in Abwesenheit eines Phasentransferkatalysators ablaufen. Der Nachteil des Verfahrens besteht darin, daß es sich auf bestimmte Fluorpyridine beschränkt und große Mengen des polar aprotischen Lösungsmittels erfordert.

In neuerer Zeit sind auch lösungsmittelfreie Verfahren bekannt geworden. Diese lösungsmittelfreien Verfahren werden in Gegenwart von Phasentransfer-Katalysatoren durchgeführt. Als besonders vorteilhaft haben sich als Katalysatoren beispielsweise quartäre Ammoniumverbindungen oder quartäre Phosphoniumverbindungen erwiesen.

Aus der EP-A-0 635 303 geht eine Katalysatormischung, die eine mindestens durch einen Alkoxypolyoxyalkyl-Rest substituierte quartäre Ammoniumverbindung und daneben ein oder mehrere quartäre Ammoniumsalze und/oder Phosphoniumsalze und/oder Polyether oder Kronenether enthalten, hervor.

Die durch mindestens einen Alkoxypolyoxyalkyl-Rest substituierten quartären Ammoniumverbindungen, die der nachfolgend genannten Komponente a) entsprechen, werden entweder alleine oder in Form der vorstehend beschriebenen Katalysatormischungen zur Herstellung von Fluornitrobenzolen (EP-A-0 635 481), zur Herstellung von mehrfach fluorierten Nitrobenzolen (EP-A-0 635 482) oder zur Herstellung von Fluornitrobenzolen (EP-A-0 635 486) verwendet.

Die durch die vorstehend beschriebenen Verfahren erzielbaren Ausbeuten sind zwar vergleichsweise gut, werden aber zum Teil durch recht lange Reaktionszeiten erkauft. Die hieraus resultierenden niedrigen Raum-Zeit-Ausbeuten stellen für ein technisches Verfahren einen großen Nachteil dar, da sie zu einer erheblichen Verteuerung der technischen Produktion führen.

Es besteht daher die Aufgabe, ein Verfahren bereitzustellen, das sich zum einen zur Herstellung einer Vielzahl fluorierter aromatischer Verbindungen eignet und sich technisch auf einfache Weise realisieren läßt und zum anderen die Nachteile langer Reaktionszeiten und niedriger Raum-Zeit-Ausbeuten vermeidet.

Diese Aufgabe wird gelöst durch ein Verfahren zur Herstellung von fluorierten Verbindungen der allgemeinen Formel (1)

$$Az_xArF_wCl_{(y-w)}R_z \qquad (1)$$

worin Az unabhängig voneinander gleich oder verschieden ist und für einen Rest -F, -Cl, -Br, $-NO_2$, -CN, $-CF_3$, $-CCl_3$, -CHO, $-CO(C_nH_{2n+1})$, wobei n eine ganze Zahl von 1 bis 10 ist, -COX oder $-SO_2X$ steht, wobei X für F, Cl oder Br steht, x eine ganze Zahl von 1 bis 3 ist, Ar einen Phenyl-Rest, Pyridyl-Rest oder Naphthylrest bedeutet, w eine ganze Zahl von 1 bis y ist, y eine ganze Zahl von 1 bis 5 bedeutet. R unabhängig voneinander gleich oder verschieden ist und für H, einen geradkettigen oder verzweigten Alkylrest mit 1 bis 10 Kohlenstoffatomen oder einen geradkettigen oder verzweigten Alkoxyrest mit 1 bis 10 Kohlenstoffatomen steht, z eine ganze Zahl von 1 bis 5 ist, wobei (x+y+z) die Zahl aller substituierbarer Valenzen am Rest Ar bedeutet.

Es ist dadurch gekennzeichnet, daß man eine Verbindung der allgemeinen Formel (2)

$$Az_xArCl_yR_z \qquad (2)$$

worin Az, x, Ar, y, R, z und (x+y+z) die gleiche Bedeutung wie in Formel 1 haben, mit einem Alkalimetallfluorid oder einem Gemisch von Alkalimetallfluoriden in Gegenwart einer Komponente a) oder einer Mischung der Komponente a) und wenigstens einer der Komponenten b), c), d) und e) umsetzt, die Komponente a) eine oder mehrere quartäre Ammoniumverbindungen der Formel (3)

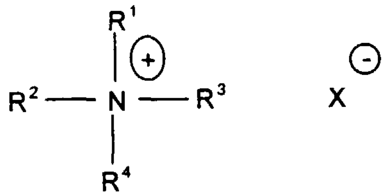

(3)

bedeutet, worin

$R^1$, $R^2$ und $R^3$ gleich oder verschieden sind und einen geradkettigen oder verzweigten Rest der Formel $-(C_mH_{2m}O)_pR^5$ bedeuten, worin $R^5$ für Wasserstoff oder einen geradkettigen oder verzweigten Alkylrest mit 1 bis 16 Kohlenstoffatomen steht, m eine ganze Zahl von 1 bis 10 und p eine Zahl von 1 bis 15 bedeuten; oder einen geradkettigen oder verzweigten Alkylrest mit 1 bis 30 Kohlenstoffatomen; oder einen substituierten Phenyl- oder Naphthylrest; oder einen substituierten Phenyl- oder Naphthylrest, wobei die Substituenten die Bedeutung Halogen, $C_1$-$C_4$-Alkyl, $C_1$-$C_4$-Alkoxy, Nitro oder Cyano haben;

$R^4$ einen geradkettigen oder verzweigten Rest der Formel $-(C_mH_{2m}O)_pR^5$ bedeutet; und

$X^{\ominus}$ ein einwertiger Säurerest oder das Äquivalent eines mehrwertigen Säurerests ist,

die Komponente b) ein oder mehrere Amidophosphoniumsalze der Formel (4)

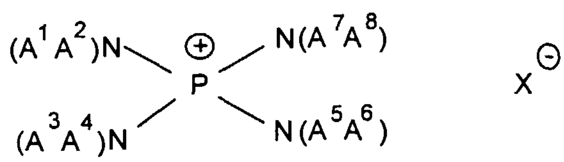

(4)

bedeutet,
worin $A^1$, $A^2$, $A^3$, $A^4$, $A^5$, $A^6$, $A^7$, $A^8$ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatomen stehen, oder $A^1A^2$, $A^3A^4$, $A^5A^6$, $A^7A^8$ unabhängig voneinander, gleich oder verschieden sind und direkt oder über O oder N-$A^9$ miteinander zu einem Ring mit 3 bis 7 Ringgliedern verbunden sind, $A^9$ für ein Alkyl mit 1 bis 4 Kohlenstoffatomen und $X^-$ einen einwertigen Säurerest oder das Äquivalent eines mehrwertigen Säurerestes ist, die Komponente c) eine oder mehrere quartäre Ammoniumverbindung der Formel (5)

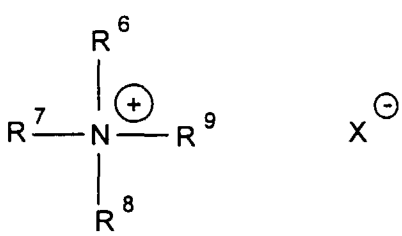

(5)

bedeutet,

$R^6$, $R^7$, $R^8$ und $R^9$ gleich oder verschieden sind und für einen geradkettigen oder verzweigten Alkylrest mit 1 bis 22 Kohlenstoffatomen; oder für einen unsubstituierten oder substituierten Arylrest oder für einen $C_1$-$C_4$-Alkyl-arylrest stehen, wobei Aryl die Bedeutung Phenyl oder Naphthyl hat und die besagten Substituenten

Halogen, $C_1$-$C_4$-Alkyl, $C_1$-$C_4$-Alkoxy, Nitro oder Cyano bedeuten; und $X^-$ ein einwertiger Säurerest oder der Äquivalent eines mehrwertigen Säurerests ist;

die Komponente d) eine oder mehrere quartäre Phosphoniumverbindungen der Formel (6)

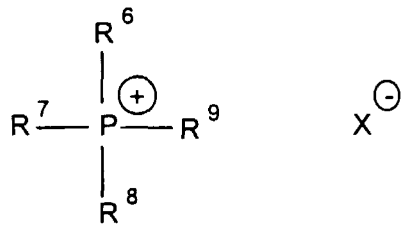 (6)

bedeutet, worin

$R^6$, $R^7$, $R^8$ und $R^9$ gleich oder verschieden sind und für einen geradkettigen oder verzweigten Alkylrest mit 1 bis 22 Kohlenstoffatomen; oder für einen unsubstituierten oder substituierten Arylrest oder für einen $C_1$-$C_4$-Alkyl-arylrest stehen, wobei Aryl die Bedeutung Phenyl oder Naphthyl hat und die besagten Substituenten Halogen, $C_1$-$C_4$-Alkyl, $C_1$-$C_4$-Alkoxy, Nitro oder Cyano bedeuten; und $X^-$ ein einwertiger Säurerest oder das Äquivalent eines mehrwertigen Säurerests ist;

die Komponente e) einen oder mehrere Polyether der Formel (7)

$$R^{10}\text{-}(O\text{-}C_aH_{2a})_b\text{-}OR^{11} \qquad (7)$$

oder Kronenether bedeutet, wobei in Formel (7)

$R^{10}$ und $R^{11}$ gleich oder verschieden sind und einen geradkettigen oder verzweigten Alkylrest mit 1 bis 16 Kohlenstoffatomen bedeuten;
a eine ganze Zahl von 2 bis 6 und
b eine ganze Zahl von 0 bis 20 ist,

daß man die Umsetzung bei 50 bis 250°C durchführt, während der Umsetzung die gebildete Verbindung der Formel (1) destillativ abtrennt und die Verbindung der Formel (2), entsprechend wie die Verbindung der Formel (1) abgetrennt wird, der Reaktionsmischung zuführt.

Das erfindungsgemäße Verfahren führt überraschenderweise zu einer deutlichen Steigerung sowohl der Raumausbeute als auch der Raum-Zeit-Ausbeute. Darüber hinaus wird in unerwarteter Weise die Bildung von unerwünschten Nebenprodukten, Folgeprodukten und/oder Zersetzungsprodukten zurückgedrängt. Die Bildung dieser Produkte hat üblicherweise eine Minderung der Ausbeute zur Folge und kann eine Reihe von Aufarbeitungsproblemen, wie Entsorgung polymerer Zersetzungsprodukte oder geringe thermische Stabilität von Produktionsrückständen, verursachen.

Besonders negativ wirken sich derartige Nebenprodukte aus, die ihrerseits zu leicht zersetzlichen Produkten mit niedrigen Zersetzungstemperaturen führen. Als Beispiel sei hierfür die Bildung von Nitrophenolen, die als Phenolate in der Reaktionssuspension vorliegen, genannt. Derartige Nebenprodukte, die recht reaktiv sind, können Auslöser für eine Reihe weiterer unerwünschter Neben- und Folgereaktionen sein, die vorzugsweise mit bereits gebildeten fluorierten aromatischen Verbindungen ablaufen. Insbesondere erweisen sich Nitrophenolate als problematisch, da sie sich, wie das Beispiel von Pikrinsäuresalzen beweist, exotherm zersetzen können.

Man kann in das erfindungsgemäße Verfahren mit gutem Erfolg eine Verbindung der Formel (2), worin Az unabhängig voneinander gleich oder verschieden ist und für einen Rest -F, -Cl, $-NO_2$, -CN, $-CF_3$, $-CCl_3$, -CHO oder $-CO(C_nH_{2n+1})$ steht, wobei n eine ganze Zahl von 1 bis 6, insbesondere 1 bis 4, vorzugsweise 1 bis 2 ist, insbesondere für einen Rest -Cl, $-NO_2$, -CN, $-CF_3$ oder -CHO steht, einsetzen.

Man kann insbesondere eine Verbindung der Formel (2), worin Ar für einen Phenyl-Rest oder Pyridyl-Rest, insbesondere für einen Phenyl-Rest steht, als geeignete Ausgangsverbindung einsetzen.

In der Verbindung der Formel (2) steht y, wie zuvor erwähnt, für eine ganze Zahl von 1 bis 5, insbesondere 1 bis 4, bevorzugt 1 bis 3, besonders bevorzugt 1 bis 2.

Man kann in das erfindungsgemäße Verfahren eine Verbindung der Formel (2) einsetzen, worin wie zuvor bereits aufgeführt, R unabhängig voneinander gleich oder verschieden ist und für H, einen geradkettigen oder verzweigten Alkylrest mit 1 bis 10 Kohlenstoffatomen oder einen geradkettigen oder verzweigten Alkoxyrest mit 1 bis 10 Kohlenstoffatomen, insbesondere für H, einen geradkettigen oder verzweigten Alkylrest mit 1 bis 4 Kohlenstoffatomen oder einen geradkettigen oder verzweigten Alkoxyrest mit 1 bis 4 Kohlenstoffatomen, bevorzugt für H, einen Alkyl- oder Alkoxyrest mit 1 bis 2 Kohlenstoffatomen, besonders bevorzugt für H steht.

Man setzt als Alkalimetallfluorid NaF, KF, RbF, CsF oder ein Gemisch derselben, insbesondere K, F oder ein Gemisch aus KF, RbF und/oder CsF ein.

Für eine Vielzahl von Fällen hat es sich als ausreichend erwiesen, als Alkalimetalllfluorid KF zu verwenden. In einigen Fällen haben sich Gemische aus KF und CsF, die 1 bis 10 Gew.-% CsF enthalten, als vorteilhaft gezeigt.

Man setzt als Katalysator üblicherweise die Komponente a) oder die Mischung der Komponente a) mit wenigstens einer der Komponenten b), c), d) und/oder e) in einer Menge von 1 bis 40, insbesondere 2 bis 30, bevorzugt 5 bis 25, besonders bevorzugt 8 bis 20 Gew.-%, bezogen auf die Verbindung der Formel (2) ein.

In einer Vielzahl von Fällen kann man die Umsetzung in Gegenwart der Komponente a) oder der Mischung von Komponente a) und wenigstens einer der Komponenten b) und d) durchführen. In diesem Falle fungiert entweder die Komponente a) oder die Mischung von a) + b) oder a) + d) oder a) + b) + d) als Katalysator.

Man setzt üblicherweise als Komponente a) eine oder mehrere quartäre Ammoniumverbindungen der Formel (3) ein, worin $R^1$, $R^2$ und $R^3$ gleich oder verschieden sind und einen linearen (geradkettigen) oder verzweigten Alkoxypolyoxyalkyl-Rest der Formel $-(C_mH_{2m}O)_pR^5$ bedeuten, worin $R^5$ für Wasserstoff oder einen geradkettigen oder verzweigten Alkylrest mit 1 bis 8 Kohlenstoffatomen, insbesondere für einen geradkettigen oder verzweigten Alkylrest mit 1 bis 8, insbesondere 1 bis 4 Kohlenstoffatomen steht, m eine ganze Zahl von 1 bis 5 und p eine ganze Zahl von 2 bis 10 bedeuten, oder einen geradkettigen oder verzweigten Alkylrest mit 1 bis 8 Kohlenstoffatomen; oder einen unsubstituierten Phenyl- oder Naphthylrest, wobei die Substituenten die Bedeutung Halogen, $C_1$-$C_4$-Alkyl, $C_1$-$C_4$-Alkoxy, Nitro, Cyano haben, bedeuten, $R^4$ einen geradkettigen oder verzweigten Rest der Formel $-(C_mH_{2m}O)_pR^5$ bedeutet, worin $R^5$ für Wasserstoff oder einen geradkettigen oder verzweigten Alkylrest mit 1 bis 8 Kohlenstoffatomen, insbesondere für einen geradkettigen oder verzweigten Alkylrest mit 1 bis 8, insbesondere 1 bis 4 Kohlenstoffatomen steht, m eine ganze Zahl von 1 bis 5 und p eine ganze Zahl von 2 bis 10 bedeuten und $X^-$ Fluorid, Chlorid, Bromid, $SO_4^{2-}/2$ oder Hydrogensulfat ist.

In dem in der Verbindung der Formel (3) enthaltenen geradkettigen oder verzweigten Alkoxypolyoxyalkyl-Rest der Formel $-(C_mH_{2m}O)_pR^5$ können gleiche oder unterschiedliche Alkoxy-Einheiten miteinander verknüpft sein. Die Anzahl der in der Verbindung der Formel (3) enthaltenen geradkettigen oder verzweigten Alkoxypolyoxyalkyl-Reste beträgt vorzugsweise 1 oder 2.

Besonders bevorzugte Verbindungen der Formel (3) im Sinne der vorliegenden Erfindung sind Dimethyl-di-(ethoxypolyoxypropyl)-ammoniumchlorid, Dimethyl-di-(ethoxypolyoxypropylmethylether)-ammoniumchlorid, Dimethyl-(ethoxypolyoxypropyl)-(ethoxypolyoxypropylmethylether)-ammoniumchlorid, Dimethyl-di-(ethoxypolyoxyethyl)-ammoniumchlorid, Dimethyl-di-(ethoxypolyoxyethylmethylether)-ammoniumchlorid, Dimethyl-(ethoxypolyoxyethyl)-(ethoxypolyoxyethylmethylether)-ammoniumchlorid, jeweils mit einer mittleren Kettenlänge p von 3, weiterhin Trimethyl-(ethoxypolyoxypropyl)-ammoniumchlorid und Trimethyl-(ethoxypolyoxypropylmethylether)-ammoniumchlorid, jeweils mit einer mittleren Kettenlänge p von 8, oder ein Gemisch der genannten Verbindungen.

Die beschriebenen Verbindungen der Formel (3) lassen sich auf bekannte Weise (US-PS 3 123 641; US-PS 3 141 905) aus den entsprechenden Ethanolaminen herstellen, die nach Umsetzung mit Alkenyloxiden und anschließender Quaternisierung mit oder ohne gleichzeitiger Veretherung in guten Ausbeuten die gewünschten Verbindungen liefern.

In der Mischung der Komponenten a) und wenigstens einer der Komponenten b), c), d) und/oder e) macht die Komponente a) 5 bis 95, insbesondere 10 bis 90, bevorzugt 20 bis 80 Gew.-% der gesamten Mischung, die als Katalysator eingesetzt wird, aus. Das molare Verhältnis von Katalysator (Komponente a) oder Gemisch von Komponente von a) und wenigstens einer der übrigen Komponenten b) bis e)) zu der Verbindung der Formel (2) kann 1:5 bis 1:150, insbesondere 1:10 bis 1:100, bevorzugt 1:20 bis 1:100 betragen. Man setzt die Verbindung der Formel (2) zu Alkalimetallfluorid in einem molaren Verhältnis von 1:(0,5 bis 3), insbesondere 1:(0,8 bis 2,5), bevorzugt von 1:(0,9 bis 1,5) ein.

Komponente b):

Man kann eine Verbindung der Formel (4), worin $A^1$, $A^2$, $A^3$, $A^4$, $A^5$, $A^6$, $A^7$, $A^8$ unabhängig voneinander, gleich oder verschieden sind und für ein geradkettiges oder verzweigtes Alkyl oder Alkenyl, insbesondere Alkyl, mit 1 bis 12, insbesondere 1 bis 8, bevorzugt 1 bis 4 Kohlenstoffatomen, oder Cycloalkyl mit 4 bis 8, insbesondere 5 bis 6 Kohlenstoffatomen, stehen, einsetzen. Diese Verbindungen sind von besonderem Interesse, da sie sich auf vergleichsweise einfache Art ausgehend von den entsprechenden Dialkylaminen, Dialkenylaminen, Dicycloalkylaminen, sekundären Aminen, die einen Alkyl- und Alkenylrest, einen Alkyl- und Cycloalkylrest oder einen Alkenyl- und Cycloalkylrest enthalten, herstellen lassen.

Man kann eine Verbindung der Formel (4), worin $A^1A^2 = A^3A^4$ oder $A^1A^2 = A^3A^4 = A^5A^6$ oder $A^1A^2 = A^3A^4 = A^5A^6 = A^7A^8$ ist, einsetzen. Diese Verbindungen, in denen zwei oder mehrere der Gruppen $A^1A^2$, $A^3A^4$, $A^5A^6$ und $A^7A^8$ einander gleich sind, sind relativ gut zugänglich.

Als Beispiele für Alkyl sind Methyl, Ethyl, n-Propyl, i-Propyl, n-Butyl, i-Butyl, n-Pentyl, 3-Methylbutyl, n-Hexyl, 2-Ethylhexyl, insbesondere Methyl, Ethyl, n-Propyl, n-Butyl und als Beispiele für Alkenyl sind Allyl, Prop-(2)-enyl, n-But-(2)-enyl, und als Beispiele für Cycloalkyl sind Cyclopentyl, Cyclohexyl, 4-Methylcyclohexyl, 4-tert.-Butylcyclohexyl, zu nennen.

Man kann auch eine Verbindung der Formel (4), worin $A^1$=$A^2$, $A^3$=$A^4$, $A^5$=$A^6$ und/oder $A^7$=$A^8$ ist, einsetzen. Diese Verbindungen sind vergleichsweise leicht zugänglich und deshalb von Interesse.

Man kann auch eine Verbindung der Formel (4), worin $A^1 = A^2 = A^3 = A^4$ oder $A^1 = A^2 = A^3 = A^4 = A^5 = A^6$ oder $A^1 = A^2 = A^3 = A^4 = A^5 = A^6 = A^7 = A^8$ ist, einsetzen.

Diese vorstehend genannten Verbindungen, in denen vier, sechs oder acht der Reste $A^1$ bis $A^8$ gleich sind, sind aufgrund ihrer leichten Zugänglichkeit ebenfalls von Interesse.

Es ist auch möglich, eine Verbindung der Formel (4), worin $A^1A^2$ oder $A^1A^2$ und $A^3$ $A^4$ oder $A^1A^2$ und $A^3A^4$ und $A^5A^6$ oder $A^1A^2$ und $A^3A^4$ und $A^5A^6$ und $A^7A^8$ direkt oder über O oder N-$A^9$ miteinander zu einem gesättigten oder ungesättigten Ring mit 5 oder 6 Ringgliedern verbunden sind, einzusetzen. Dementsprechend enthalten diese Verbindungen einen, zwei, drei oder vier der vorstehend erwähnten Ringe.

Es ist ferner möglich, eine Verbindung der Formel (4), worin $A^1A^2$ oder $A^1A^2$ und $A^3$ $A^4$ oder $A^1A^2$ und $A^3A^4$ und $A^5A^6$ oder $A^1A^2$ und $A^3A^4$ und $A^5A^6$ und $A^7A^8$ zu einem Ring, der das N-Atom, an dem die jeweiligen Reste $A^1$ bis $A^8$ sitzen, gegebenenfalls O oder N-$A^9$ und $CH_2$-Gruppen als Ringglieder umfaßt, verbunden sind, einzusetzen. In dieser Stoffgruppe bilden das N-Atom mit den an ihnen jeweils befindlichen Resten $A^1$ bis $A^8$ beispielsweise einen Hexahydropyridinring (Piperidinring), Tetrahydropyrrolring (Pyrrolidinring), einen Hexahydropyrazinring (Piperazinring) oder Morpholinring.

Dementsprechend enthalten diese Verbindungen einen, zwei, drei oder vier der vorstehend erwähnten Ringe.

In den Verbindungen der Formeln (3), (4), (5) und (6) steht $X^-$, wie bereits eingangs erwähnt, für einen einwertigen Säurerest oder das Äquivalent eines mehrwertigen Säurerestes, insbesondere den Rest einer anorganischen Mineralsäure, einer organischen Carbonsäure, einer aliphatischen oder aromatischen Sulfonsäure.

Üblicherweise setzt man eine Verbindung der Formel (3), (4), (5) und (6) worin $X^-$ für $F^-$, $Cl^-$, $Br^-$, $J^-$, $HF_2^-$, $BF_4^-$, $C_6H_5SO_3^-$, p-$CH_3$-$C_6H_5SO_3^-$, $SO_4^{2-}/2$, $HSO_4^-$, $PF_6^-$, $CF_3SO_3^-$, insbesondere für $F^-$, $Cl^-$, $Br^-$, $J^-$, $HF_2^-$, $BF_4^-$, steht, ein.

Als Komponente b) kann man ein oder mehrere Amidophosphoniumsalze der Formel (4), worin drei Reste $(A^1A^2)N$, $(A^3A^4)N$ und $(A^5A^6)N$ gleich sind und für einen Dialkylaminorest mit 1 bis 6, insbesondere 1 bis 4 Kohlenstoffatomen je Alkyl, der Dialkylaminorest insbesondere zwei gleiche Alkylgruppen enthält, oder für einen Pyrrolidin-, Piperidin- oder Morpholinring stehen, der Rest $(A^7A^8)N$ sich von den vorstehend genannten Resten unterscheidet, wobei $A^7$ und $A^8$ gleich oder verschieden sind und für einen Alkylrest mit 1 bis 8 Kohlenstoffatomen oder einen Alkenylrest mit 1 bis 4 Kohlenstoffatomen stehen, oder alle vier Reste $(A^1A^2)N$, $(A^3A^4)N$, $(A^5A^6)N$ und $(A^7A^8)N$ gleich sind und für einen Dialkylaminorest mit 1 bis 6, insbesondere 1 bis 4, bevorzugt 1 bis 2 Kohlenstoffatomen je Alkyl oder für einen Pyrolidin-, Piperidin- oder Morpholin-Ring stehen, einsetzen.

Ohne Anspruch auf Vollständigkeit zu erheben, seien als Beispiele für Verbindungen der Formel (4) genannt:

Tetrakis(dimethylamino)phosphoniumchlorid
Tetrakis(diethylamino)phosphoniumchlorid
Tetrakis(dimethylamino)phosphoniumbromid
Tetrakis(diethylamino)phosphoniumbromid
Tetrakis(dipropylamino)phosphoniumchlorid oder -bromid
Tris(diethylamino)(dimethylamino)phosphoniumchlorid oder -bromid
Tetrakis(dibutylamino)phosphoniumchlorid oder -bromid
Tris(dimethylamino)(diethylamino)phosphoniumchlorid oder -bromid
Tris(dimethylamino)(cyclopentylamino)phosphoniumchlorid oder -bromid
Tris(dimethylamino)(dipropylamino)phosphoniumchlorid oder -bromid
Tris(dimethylamino)(dibutylamino)phosphoniumchlorid oder -bromid
Tris(dimethylamino)(cyclohexylamino)phosphoniumchlorid oder -bromid
Tris(dimethylamino)(diallylamino)phosphoniumchlorid oder -bromid
Tris(dimethylamino)(dihexylamino)phosphoniumchlorid oder -bromid
Tris(diethylamino)(dihexylamino)phosphoniumchlorid oder -bromid
Tris(dimethylamino)(diheptylamino)phosphoniumchlorid oder -bromid
Tris(diethylamino)(diheptylamino)phosphoniumchlorid oder -bromid
Tetrakis(pyrrolidino)phosphoniumchlorid oder -bromid

Tetrakis(piperidino)phosphoniumchlorid oder -bromid
Tetrakis(morpholino)phosphoniumchlorid oder -bromid
Tris(piperidino)(diallylamino)phosphoniumchlorid oder -bromid
Tris(pyrrolidino)(ethylmethylamino)phosphoniumchlorid oder -bromid
Tris(pyrrolidino)(diethylamino)phosphoniumchlorid oder -bromid.

Man kann auch ein Gemisch zweier oder mehrerer Verbindungen der Formel (4) verwenden. Besonders einfach gestaltet sich dies, wenn man Gemische von Verbindungen der Formel (4) verwendet.

Die Verbindungen der Formel (4) lassen sich beispielsweise durch Umsetzung von Phosphorpentachlorid mit Dialkylaminen herstellen. Aus der nachfolgenden Gleichung ist die Umsetzung unter Verwendung von Dimethylamin zu entnehmen:

$$PCl_5 + HN(CH_3)_2 \rightarrow P[N(CH_3)_2]_4\ Cl$$

Man kann aber auch Phosphorpentachlorid stufenweise mit unterschiedlichen sekundären Aminen, beispielsweise Dialkylaminen, zur Reaktion bringen, um unsymmetrisch substituierte Verbindungen der Formel (4) zu erhalten. Weitere Möglichkeiten, Verbindungen der Formel (4) zu synthetisieren, sind von R. Schwesinger et al., Angew. Chem. 103 (1991) 1376 und R. Schwesinger et al., Chem. Ber. 127 (1994) 2435 bis 2454 beschrieben.

Es hat sich bewährt, als Komponente c) ein oder mehrere quartäre Ammoniumverbindungen der Formel (5), worin $R^6$, $R^7$ und $R^8$ gleich sind und für einen Alkylrest mit 1 bis 4, insbesondere 1 bis 2 Kohlenstoffatomen stehen und $R^9$ ein Alkylrest mit 6 bis 24, insbesondere 8 bis 20, bevorzugt 10 bis 18 Kohlenstoffatomen ist oder $R^6$, $R^7$, $R^8$ und $R^9$ gleich sind und einen Alkylrest mit 1 bis 6, insbesondere 1 bis 4 Kohlenstoffatomen bedeuten, einsetzt.

Ohne Anspruch auf Vollständigkeit zu erheben seien als einige Vertreter der Komponente c) Octadecyltrimethylammoniumchlorid, Hexadecyltrimethylammoniumchlorid, Benzyltrimethylammoniumchlorid, Tetramethylammoniumchlorid, Tetramethylammoniumbromid, Tetraethylammoniumchlorid , Tetraethylammoniumbromid, Tetrabutylammoniumchlorid und Tetrabutylammoniumbromid genannt. Es lassen sich auch Gemische der vorstehenden Verbindungen verwenden.

Es hat sich ferner als günstig erwiesen, als Komponente d) ein oder mehrere quartäre Phosphoniumverbindungen der Formel (6), worin $R^6$, $R^7$ und $R^8$ gleich sind und für einen Alkylrest mit 1 bis 4, insbesondere 1 bis 2 Kohlenstoffatomen stehen und $R^9$ ein Alkylrest mit 6 bis 24, insbesondere 8 bis 20, bevorzugt 10 bis 18 Kohlenstoffatomen ist, oder $R^6$, $R^7$, $R^8$ und $R^9$ gleich sind und für einen Phenylrest oder einen Alkylrest mit 1 bis 6, insbesondere 1 bis 4 Kohlenstoffatomen bedeuten, einzusetzen.

Als Beispiele für geeignete Verbindungen der Komponente d) seien Hexadecyltributylphosphoniumbromid, Stearyltributylphosphoniumbromid, Tetrabutylphosphoniumchlorid, Tetrabutylphosphoniumbromid, Tetraoctylphosphoniumbromid, Tetraphenylphosphoniumchlorid und Tetraphenylphosphoniumbromid erwähnt. Es lassen sich auch Gemische der vorstehenden Verbindungen verwenden.

In einer Reihe von Fällen ist es von Vorteil, als Komponente e) einen oder mehrere Polyether der Formel (7), worin $R^{10}$ und $R^{11}$ gleich oder verschieden sind und einen geradkettigen oder verzweigten Alkylrest mit 1 bis 8, insbesondere 1 bis 6 Kohlenstoffatomen bedeuten, a eine ganze Zahl von 2 bis 3 und b eine ganze Zahl von 4 bis 14, insbesondere 4 bis 8 ist, oder einen Kronenether, insbesondere einen Polyether der Formel (7), worin $R^{10}$ und $R^{11}$ gleich sind und einen Alkylrest mit 1 bis 8, insbesondere 1 bis 6 Kohlenstoffatomen bedeuten, a eine ganze Zahl von 2 bis 3 und b eine ganze Zahl von 4 bis 14, insbesondere 6 bis 10 ist, einzusetzen.

Von Interesse sind Gemische der Komponente a), b), c), d) und e) wenn a):b) im Gewichtsverhältnis 10:1 bis 1:10, insbesondere 5:1 bis 1:5 und die Komponenten c), d) und e) in einer Menge von 0 bis 25, insbesondere 5 bis 20 Gew.-%, bezogen auf die Mischung enthalten sind.

In einer Vielzahl von Fällen hat sich eine Mischung der Komponenten a), b) und d), worin a) : b) : d) im Gewichtsverhältnis (10 bis 1):(5 bis 0,1) : (5 bis 0,1), insbesondere (8 bis 2) : (3 bis 0,5) : (3 bis 0,5) enthalten sind, als günstig erwiesen.

Mischungen der Komponenten a) und d), worin a) : d) im Gewichtsverhältnis 10:1 bis 1:10, insbesondere 5:1 bis 1:5 enthalten sind, lassen sich auch mit Erfolg einsetzen.

Man führt die Umsetzung der Verbindung der Formel (2), wie eingangs bereits erwähnt, bei 50 bis 250, insbesondere 70 bis 220, bevorzugt 100 bis 200°C durch.

Es stellt einen weiteren Vorteil dar, daß man das erfindungsgemäße Verfahren in Abwesenheit eines Lösungsmittels, insbesondere in Abwesenheit eines dipolar aprotischen Lösungsmittels durchführen kann. Beispiele für dipolar aprotische Lösungsmittel sind Dimethylsulfoxid, Dimethylformamid, Diethylformamid, Dimethylacetamid, Diethylacetamid, Hexamethylphosphorsäuretriamid, Tetramethylharnstoff, Sulfolan (Tetramethylensulfon) und N-Methylpyrrolidon. Das erfindungsgemäße Verfahren eröffnet in vorteilhafter Weise einen Weg, auf die Verwendung solcher Lösungsmittel, die üblicherweise in großen Mengen, bezogen auf das Ausgangsmaterial, eingesetzt werden, zu verzichten.

Dadurch kann das für die Umsetzung zur Verfügung stehende Reaktorvolumen wesentlich besser ausgenutzt werden.

Man wählt die Reaktionsbedingungen derart, daß die gebildete fluorierte Verbindung der Formel (1) siedet und abdestilliert werden kann, die während der Umsetzung zugesetzte Ausgangsverbindung der Formel (2) hingegen nicht siedet, sondern im Reaktionsgemisch bleibt. In einer Vielzahl von Fällen empfiehlt es sich, die Umsetzung bei einem Druck von 0,001 bis 1, insbesondere 0,005 bis 0,8, bevorzugt 0,01 bis 0,7 bar durchzuführen.

Man kann die gebildete Verbindung der Formel (1) absatzweise oder kontinuierlich aus dem Reaktionsgemisch abdestillieren. Besonders günstig ist es, die gebildete Verbindung der Formel (1) kontinuierlich aus dem Reaktionsgemisch abzudestillieren.

Man kann das Ausgangsmaterial, also die Verbindung der Formel (2), der Reaktionsmischung absatzweise, also verteilt auf mehrere gleich große oder verschiedene große Portionen, zusetzen oder die Zugabe der Verbindung der Formel (2) kontinuierlich vornehmen. Die kontinuierliche Zugabe der Verbindung der Formel (2) ist besonders vorteilhaft.

Dadurch, daß man die Verbindung der Formel (2), also das Ausgangsmaterial der Reaktionsmischung - entsprechend wie das Endprodukt der Formel (1) abgetrennt wird - zuführt, erreicht man, daß das Volumen des Reaktionsgemisches annähernd konstant gehalten wird und keinen größeren Schwankungen unterliegt. Die Abweichungen des in Reaktion befindlichen Volumens betragen während der Umsetzung üblicherweise ± 20, insbesondere ± 10 Vol.-%, bezogen auf das Reaktionsgemisch.

Die nachfolgenden Beispiele beschreiben die vorliegende Erfindung näher, ohne sie zu beschränken.

Experimenteller Teil

Beispiel 1: Herstellung von 2-Chlor-4,5-difluornitrobenzol

In einem 2,5-Liter Planschiffkolben mit Destillationsbrücke und Ankerrührer werden bei 87°C, in eine Schmelze von 1680 g (8,0 mol) 2,4-Dichlor-5-fluornitrobenzol und 202 g (0,3 mol) Methyl - tris - (methyltetraethoxy) - ammoniumchlorid, 836,6 g (14,4 mol) Kaliumfluorid eingetragen. Anschließend werden 300 g (2,8 mol) Xylol zugegeben und die Reaktionssuspension durch Anlegen eines Vakuums von 20 mbar und einer Temperaturerhöhung auf 100°C azeotrop getrocknet.

Das Vakuum wird auf 50 mbar reduziert, die Reaktionstemperatur auf 128 bis 130°C angehoben und die Reaktionssuspension für 11,5 Stunden unter diesen Bedingungen gehalten. Während dieser Zeit wird kontinuierlich Destillat abgenommen (115 bis 118°C Kopftemperatur) und gleichzeitig die entsprechende Menge an 2,4-Dichlor-5-fluornitrobenzol (insgesamt: 1750 g (8,3 mol)) unter guter Rührung zudosiert.

Nach dem Reaktionsende wird die Reaktionssuspension auf 25°C gekühlt und scharf abgesaugt (25°C). Die abgetrennten Salze werden dreimal mit insgesamt 450 g Xylol nachgewaschen und die vereinigten organischen Phasen fraktioniert destilliert.

Ausbeute:

920 g (4,75 mol) 2-Chlor-4,5-difluornitrobenzol, neben 1550 g (7,53 mol) nicht umgesetztem 2,4-Dichlor-5-fluornitrobenzol; das entspricht einer Ausbeute von 29,1 %, bezogen auf eingesetztes 2,4-Dichlor-5-fluornitrobenzol, und 54,2 %, bezogen auf umgesetztes 2,4-Dichlor-5-fluornitrobenzol.

| | |
|---|---|
| Raumausbeute: | 368 g/l |
| Raum-Zeitausbeute: | 32,0 g/lxh (Gramm/Liter x Stunde) |

Vergleichsbeispiel 1: Herstellung von 2-Chlor-4,5-difluornitrobenzol

In einem 2,5-Liter Planschliffkolben mit Destillationsbrücke und Ankerrührer werden bei 87°C, in eine Schmelze von 1680 g (8,0 mol) 2,4-Dichlor-5-fluornitrobenzol und 202 g (0,3 mol) Methyl - tris - (methyltetraethoxy) - ammoniumchlorid, 557,7 g (9,6 mol) Kaliumfluorid eingetragen. Anschließend werden 300 g (2,8 mol) Xylol zugegeben und die Reaktionssuspension durch Anlegen eines Vakuums von 10 mbar und einer Temperaturerhöhung auf 120°C azeotrop getrocknet. Sind die 120°C erreicht und kein Xylol destilliert mehr über, wird die Destillationsbrücke gegen einen Rückflußkühler ausgetauscht und die Reaktionssuspension für 11,5 Stunden unter gutem Rühren bei dieser Temperatur gehalten. Anschließend wird die Reaktionssuspension auf 25°C gekühlt und scharf abgesaugt (25°C). Die abgetrennten Salze werden dreimal mit insgesamt 450 g Xylol nachgewaschen und die vereinigten organischen Phasen fraktioniert.

Umsatz: 69 GC-FL %

Ausbeute:

420 g (2,17 mol) 2-chlor-4,5-difluornitrobenzol, neben 490 g (2,36 mol) nicht umgesetztem 2,4-Dichlor-5-fluornitrobenzol; das entspricht einer Ausbeute von 27,1 %, bezogen auf eingesetztes 2,4-Dichlor-5-fluornitrobenzol, und 38,3 %, bezogen auf umgesetztes 2,4-Dichlor-5-fluornitrobenzol.

Raumausbeute: 168 g/l
Raum-Zeitausbeute: 14,6 g/lxh

Beispiel 2: Herstellung von 2-Fluornitrobenzol

In einem 2,5-Liter Planschliffkolben mit Destillationsbrücke und Ankerrührer werden bei 87°C, in eine Schmelze von 1575 g (10,0 mol) 2-Chlornitrobenzol und 315,0 g (0,5 mol) Methyl - tris - (methyltetraethoxy) - ammoniumchlorid, 61,0 g (0,18 mol) Tetrabutylphosphoniumbromid und 1007,5 g (17,3 mol) Kaliumfluorid eingetragen. Anschließend werden 260 g (2,8 mol) Xylol zugegeben und die Reaktionssuspension durch Anlegen eines Vakuums von 20 mbar und einer Temperaturerhöhung auf 100°C azeotrop getrocknet.

Das Vakuum wird auf ca. 90 bis 100 mbar reduziert, die Reaktionstemperatur auf 158 bis 164°C angehoben und die Reaktionssuspension für 10 Stunden unter diesen Bedingungen gehalten. Während dieser Zeit wird kontinuierlich Destillat abgenommen (80 bis 145°C Kopftemperatur) und gleichzeitig die entsprechende Menge an 2-Chlornitrobenzol (insgesamt: 1947 g (12,4 mol)) unter guter Rührung zudosiert.

Nach dem Reaktionsende wird die Reaktionssuspension auf 25°C gekühlt und scharf abgesaugt (25°C). Die abgetrennten Salze werden dreimal mit insgesamt 450 g Xylol nachgewaschen und die erhaltene Mutterlauge ohne weitere Reinigung mit 869,4 g (15,0 mol) frischem Kaliumfluorid versetzt. Anschließend erfolgt die weitere Reaktionsführung wie zuvor beschrieben. Azeotrope Trocknung (100°C, 20 mbar), nachfolgend 157 bis 160°C, 90 mbar, semi - batch Fahrweise, Reaktionszeit 5,5 Stunden.

Die so erhaltene Mutterlauge wird entsprechend der oben beschriebenen Prozedur, filtriert, mit Xylol gewaschen und anschließend zusammen mit den vereinigten Destillaten fraktioniert destilliert.

Ausbeute:

1772 g (12,6 mol) 2-Fluornitrobenzol, neben 2481 g (15,8 mol) nicht umgesetztem 2-Chlornitrobenzol; das entspricht einer Ausbeute von 42,9 %, bezogen auf eingesetztes 2-Chlornitrobenzol, und 93,0 %, bezogen auf umgesetztes 2-Chlornitrobenzol.

Raumausbeute[1]: 490 g/l
Raum-Zeitausbeute: 50,1 g/lxh

Vergleichsbeispiel 2: Herstellung von 2-Fluornitrobenzol

In einem 2,5 l Planschliffkolben mit Destillationsbrücke und Impellerrührer werden bei 65°C, in eine Schmelze von 1890 g (12,0 mol) 2-Chlornitrobenzol, 113,4 g (0,1 mol) Methyl - tris - (methyltetraethoxy) - ammoniumchlorid, 56,7 g (0,17 mol) Tetrabutylphosphoniumbromid und 697,2 g (12,0 mol) Kaliumfluorid eingetragen. Anschließend wird mit 150 g (1,41 mol) Xylol bis 120°C unter verminderten Druck azeotrop getrocknet, die Reaktionssuspension auf 170°C aufgeheizt und für 21 Stunden unter guter Rührung bei dieser Temperatur gehalten.

Umsatz: 79 GC-FL %
Ausbeute:

1085 g (7,7 mol) 2-Fluornitrobenzol, neben 315 g (2,0 mol) nicht umgesetztem 2-Chlornitrobenzol; das entspricht einer Ausbeute von 64,2 %, bezogen auf eingesetztes 2-Chlornitrobenzol, und 77,0 %, bezogen auf umgesetztes 2-Chlornitrobenzol

Raumausbeute: 434 g/l
Raum-Zeitausbeute: 20,7 g/lxh

[1] 1227 g (8,7 mol) im ersten Konti-Ansatz

Beispiel 3: Herstellung von 2-Chlor-6-fluorbenzaldehyd

In einem 2,5-Liter Planschliffkolben mit Destillationsbrücke und Impellerrührer werden bei 80°C, in eine Schmelze von 875 g (5,0 mol) 2,6-Dichlorbenzaldehyd und 87 g (0,13 mol) Methyl - tris - (methyltetraethoxy) - ammoniumchlorid, 290 g (5,0 mol) Kaliumfluorid eingetragen. Anschließend werden 22 g (0,055 mol) Tetrakis(diethylamino)phosphonium - Bromid eingetragen und 66 g (0,6 mol) Chlorbenzol zugefügt und die Reaktionssuspension durch Anlegen eines Vakuums von 20 mbar und einer Temperaturerhöhung auf 127°C Innentemperatur azeotrop getrocknet.
Das Vakuum wird nach der Azeotroptrocknung auf 55 mbar zurückgenommen, die Reaktionstemperatur auf 168 bis 170°C Innentemperatur erhöht und die Reaktionssuspension 20 Stunden unter diesen Bedingungen gehalten. Während dieser Zeit wird kontinuierlich Destillat abgenommen (120 bis 130°C Kopftemperatur) und gleichzeitig werden im Verlauf der Reaktion weitere 263 g (1,5 mol) 2,6-Dichlorbenzaldehyd zudosiert. Es fallen während der 20 Stunden 560 g Destillat an.
Nach Reaktionsende wird die zurückbleibende Reaktionssuspension bei 100°C unter Rühren mit 500 ml Chlorbenzol versetzt und bei 25°C scharf abgesaugt. Die abgetrennten Salze werden dreimal mit insgesamt 500 ml Chlorbenzol nachgewaschen und sämtliche organischen Phasen durch fraktionierte Destillation gereinigt.

Ausbeute:

443 g (2,79 mol) 2-Chlor-6-fluorbenzaldehyd neben 446 g (2,55 mol) nicht umgesetztem 2,6-Dichlorbenzaldehyd; dies entspricht einer Ausbeute von 42,9 % d.Th., bezogen auf eingesetzten 2,6-Dichlorbenzaldehyd, und 70,6 % d.Th., bezogen auf umgesetzten 2,6-Dichlorbenzaldehyd.
Weiterhin werden nach Fraktionierung nur noch 29 g (0,20 mol) 2,6-Difluorbenzaldehyd isoliert (3,1 Mol%),

Raumausbeute: 177 g/l
Raum-Zeitausbeute : 8,9 g/lxh

Vergleichsbeispiel 3: Herstellung von 2-Chlor-6-fluorbenzaldehyd

In einem 2,5-Liter-Planschliffkolben mit Destillationsbrücke und Impellerrührer werden bei ca. 80 bis 85°C, in eine Schmelze von 875 g (5,0 mol) 2,6-Dichlorbenzaldehyd und 87 g (0,13 mol) Methyl-tris-(methyltetraethoxy)-ammoniumchlorid, 290 g (5 mol) Kaliumfluorid eingetragen. Anschließend werden 22 g (0,055 mol) Tetrakis(diethylamino)phosphonium-Bromid eingetragen und sodann werden 66 g (0,6 mol) Chlorbenzol zugefügt und die Reaktionssuspension durch Anlegen eines Vakuums von 20 mbar und einer Temperaturerhöhung auf 127°C Innentemperatur azeotrop getrocknet. Sobald die Innentemperatur 127°C überschreitet und kein Chlorbenzol weiter überdestilliert, wird das Vakuum aufgehoben und die Destillationsbrücke gegen einen Rückflußkühler ausgetauscht.
Der Ansatz wird über 21 Stunden unter Schutzgas bei 170°C gerührt. Anschließend wird die Reaktionssuspension auf 60°C gekühlt und scharf abgesaugt. Die abgetrennten Salze werden fünfmal mit insgesamt 500 ml Chlorbenzol gewaschen und die vereinigten organischen Phasen fraktioniert.

Umsatz: 71,5 GC-Fl.%

Ausbeute:

314 g (1,98 mol) 2-Chlor-6-fluorbenzaldehyd, neben 225 g (1,29 mol) nicht umgesetztem 2,6-Dichlorbenzaldehyd; dies entspricht einer Ausbeute von 39,6 % d.Th., bezogen auf eingesetzten 2,6-Dichlorbenzaldehyd, und 53,4 % d.Th., bezogen auf umgesetzten 2,6-Dichlorbenzaldehyd.
Weiterhin werden nach Fraktionierung
194 g (0,73 mol) 2,6-Difluorbenzaldehyd isoliert (14,6 Mol%)

Raumausbeute: 126 g/l
Raum-Zeitausbeute: 6,0 g/lxh

Vergleichsbeispiel 3a: Herstellung von 2-Chlor-6-fluorbenzaldehyd

Man arbeitet wie im Vergleichsbeispiel 3, setzt aber 875 g (5,0 mol) 2,6-Dichlorbenzaldehyd mit 232 g (4 mol) Kaliumfluorid, entsprechend 80 Mol-% um. Die Reaktion wird über GC-Umsatzkontrolle verfolgt und wird bereits nach 18 Stunden abgebrochen, weil kein weiterer Umsatz stattfindet. Die Produktisolierung erfolgt durch fraktionierte Destillation.

Umsatz: 60,5 GC-Fl. %

Ausbeute:

282 g (1,77 mol) 2-Chlor-6-fluorbenzaldehyd, neben 346 g (1,98 mol) nicht umgesetztem 2,6-Dichlorbenzaldehyd; dies entspricht einer Ausbeute von 35,4 % d.Th., bezogen auf eingesetzten 2,6-Dichlorbenzaldehyd und 58,6 % d.Th., bezogen auf umgesetzten 2,6-Dichlorbenzaldehyd.

Weiterhin werden nach Fraktionierung 54,3 g (0,38 mol) 2,6-Difluorbenzaldehyd isoliert (7,6 Mol%).

Raumausbeute: 113 g/l
Raum-Zeitausbeute: 6,3 g/lxh

Beispiel 4: Herstellung von 4-Chlor-2,3-difluornitrobenzol

In einem 2,5-Liter Planschliffkolben mit Destillationsbrücke und Ankerrührer werden bei 68°C, in eine Schmelze von 1679,2 g (8,0 mol) 2,4-Dichlor-3-fluornitrobenzol und 335,8 g (0,53 mol) Methyl-tris-(methyltetraethoxy)-ammoniumchlorid, 1047,4 g (18,03 mol) Kaliumfluorid eingetragen. Anschließend werden 205 g (1,93 mol) Xylol zugegeben und die Reaktionssuspension durch Anlegen eines Vakuums von 20 mbar und einer Temperaturerhöhung auf 130°C azeotrop getrocknet.

Das Vakuum wird auf 30 mbar reduziert, die Reaktionstemperatur auf 137 bis 145°C angehoben und die Reaktionssuspension für 4,5 Stunden unter diesen Bedingungen gehalten. Während dieser Zeit wird kontinuierlich Destillat abgenommen (122 bis 132°C Kopftemperatur) und gleichzeitig die entsprechende Menge an 2,4-Dichlor-3-fluornitrobenzol (insgesamt: 1050 g (5,0 mol)) unter gutem Rühren zudosiert.

Nach dem Reaktionsende wird die Reaktionssuspension auf 25°C gekühlt und scharf abgesaugt (25°C). Die abgetrennten Salze werden dreimal mit insgesamt 450 g Xylol nachgewaschen und die vereinigten organischen Phasen fraktioniert destilliert.

Ausbeute:

655 g (3,38 mol) 4-Chlor-2,3-difluornitrobenzol, neben 1463 g (6,97 mol) nicht umgesetztem 2,4-Dichlor-3-fluornitrobenzol; das entspricht einer Ausbeute von 26,0 %, bezogen auf eingesetztes 2,4-Dichlor-3-fluornitrobenzol, und
56,1 %, bezogen auf umgesetztes 2,4-Dichlor-3-fluornitrobenzol.

Raumausbeute: 262 g/l
Raum-Zeitausbeute: 58,2 g/lxh

Beispiel 5: Herstellung von 2-Chlor-4,5-difluornitrobenzol

In einem 2,5-Liter Planschliffkolben mit Destillationsbrücke und Ankerrührer werden bei 87°C, in die Schmelze von 1680 g (8,0 mol) 2,4-Dichlor-5-fluornitrobenzol und 336,0 g (0,53 mol) Methyl-tris-(methyltetraethoxy)-ammoniumchlorid, 836,6 g (14,4 mol) Kaliumfluorid eingetragen. Anschließend werden 150 g (2,8 mol) Xylol zugegeben und die Reaktionssuspension durch Anlegen eines Vakuums von 20 mbar und einerTemperaturerhöhung auf 100°C azeotrop getrocknet.

Das Vakuum wird auf 30 mbar reduziert, die Reaktionstemperatur auf 128 bis 130°C angehoben und die Reaktionssuspension für 6,5 Stunden unter diesen Bedingungen gehalten. Während dieser Zeit wird kontinuierlich Destillat abgenommen (110 bis 115°C Kopftemperatur) und gleichzeitig die entsprechende Menge an 2,4-Dichlor-5-fluornitrobenzol (insgesamt: 1200 g (5,7 mol)) unter guter Rührung zudosiert.

Nach dem Reaktionsende wird die Reaktionssuspension auf 25°C gekühlt und scharf abgesaugt. Die abgetrennten Salze werden dreimal mit insgesamt 450 g Xylol nachgewaschen und die erhaltene Mutterlauge ohne weitere Reinigung mit 836,6 g (14,4 mol) frischem Kaliumfluorid versetzt. Anschließend erfolgt die weitere Reaktionsführung wie zuvor beschrieben. Azeotrope Trocknung (100°C, 20 mbar), nachfolgend 128 bis 130°C, 30 mbar, semi - batch Fahrweise, Reaktionszeit 5,5 Stunden.

Die so erhaltene Mutterlauge wird entsprechend der oben beschriebenen Prozedur, filtriert, mit Xylol gewaschen und anschließend zusammen mit den vereinigten Destillaten fraktioniert destilliert.

Ausbeute:

1112 g (5,8 mol) 2-Chlor-4,5-difluornitrobenzol, neben 1150 g (5,5 mol) nicht umgesetztem 2,4-Dichlor-5-fluornitrobenzol; das entspricht einer Ausbeute von 31,4 %, bezogen auf eingesetztes 2,4-Dichlor-5-fluornitrobenzol, und 44,6 %, bezogen auf umgesetztes 2,4-Dichlor-5-fluornitrobenzol.

| | |
|---|---|
| Raumausbeute: | 444,8 g/l |
| Raum-Zeitausbeute: | 38,7 g/lxh |

## Patentansprüche

1. Verfahren zur Herstellung von fluorierten Verbindungen der allgemeinen Formel (1)

$$Az_xArF_wCl_{(y-w)}R_z \qquad (1)$$

worin Az unabhängig voneinander gleich oder verschieden ist und für einen Rest -F, -Cl, -Br, $-NO_2$, -CN, $-CF_3$, $-CCl_3$, -CHO, $-CO(C_nH_{2n+1})$, wobei n eine ganze Zahl von 1 bis 10 ist, -COX oder $-SO_2X$ steht, wobei X für F, Cl oder Br steht, x eine ganze Zahl von 1 bis 3 ist, Ar einen Phenyl-Rest, Pyridyl-Rest oder Naphthylrest bedeutet, w eine ganze Zahl von 1 bis y ist, y eine ganze Zahl von 1 bis 5 bedeutet, R unabhängig voneinander gleich oder verschieden ist und für H, einen geradkettigen oder verzweigten Alkylrest mit 1 bis 10 Kohlenstoffatomen oder einen geradkettigen oder verzweigten Alkoxyrest mit 1 bis 10 Kohlenstoffatomen steht, z eine ganze Zahl von 1 bis 5 ist, wobei ( x+y+z ) die Zahl aller substituierbarer Valenzen am Rest Ar bedeutet, dadurch gekennzeichnet, daß man eine Verbindung der allgemeinen Formel (2)

$$Az_xArCl_yR_z \qquad (2),$$

worin Az, x, Ar, y, R, z und ( x+y+z ) die gleiche Bedeutung wie in Formel 1 haben, mit einem Alkalimetallfluorid oder einem Gemisch von Alkalimetallfluoriden in Gegenwart einer Komponente a) oder einer Mischung der Komponente a) und wenigstens einer der Komponenten b), c), d) und e) umsetzt, die Komponente a) eine oder mehrere quartäre Ammoniumverbindungen der Formel (3)

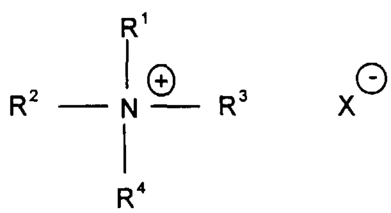

(3)

bedeutet, worin

| | |
|---|---|
| $R^1$, $R^2$ und $R^3$ | gleich oder verschieden sind und einen geradkettigen oder verzweigten Rest der Formel $-(C_mH_{2m}O)_pR^5$ bedeuten, worin $R^5$ für Wasserstoff oder einen geradkettigen oder verzweigten Alkylrest mit 1 bis 16 Kohlenstoffatomen steht, m eine ganze Zahl von 1 bis 10 und p eine Zahl von 1 bis 15 bedeuten; oder einen geradkettigen oder verzweigten Alkylrest mit 1 bis 30 Kohlenstoffatomen; oder einen substituierten Phenyl- oder Naphthylrest; oder eine substituierten Phenyl- oder Naphthylrest, wobei die Substituenten die Bedeutung Halogen, $C_1$-$C_4$-Alkyl, $C_1$-$C_4$-Alkoxy, Nitro oder Cyano haben; |
| $R^4$ | einen geradkettigen oder verzweigten Rest der Formel $-(C_mH_{2m}O)_pR^5$ bedeutet; und |
| $X^{\ominus}$ | ein einwertiger Säurerest oder das Äquivalent eines mehrwertigen Säurerests ist, |

die Komponente b) ein oder mehrere Amidophosphoniumsalze der Formel (4)

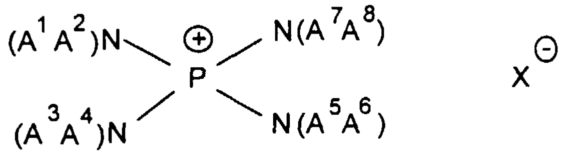

(4)

bedeutet,
worin $A^1$, $A^2$, $A^3$, $A^4$, $A^5$, $A^6$, $A^7$, $A^8$ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatomen stehen, oder $A^1A^2$, $A^3A^4$, $A^5A^6$, $A^7A^8$ unabhängig voneinander, gleich oder verschieden sind und direkt oder über O oder N-$A^9$ miteinander zu einem Ring mit 3 bis 7 Ringgliedern verbunden sind, $A^9$ für ein Alkyl mit 1 bis 4 Kohlenstoffatomen und $X^-$ein einwertiger Säurerest oder das Äquivalent eines mehrwertigen Säurerestes ist,
die Komponente c) eine oder mehrere quartäre Ammoniumverbindungen der Formel (5)

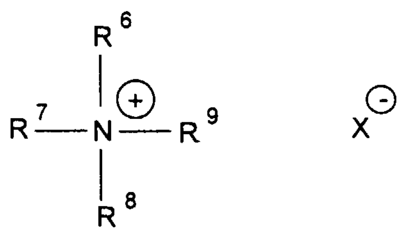

(5)

bedeutet,

$R^6$, $R^7$, $R^8$ und $R^9$ gleich oder verschieden sind und für einen geradkettigen verzweigten Alkylrest mit 1 bis 22 Kohlenstoffatomen;
oder für einen unsubstituierten oder substituierten Arylrest oder für einen $C_1$-$C_4$-Alkyl-arylrest stehen, wobei Aryl die Bedeutung Phenyl oder Naphthyl hat und die besagten Substituenten Halogen, $C_1$-$C_4$-Alkyl, $C_1$-$C_4$-Alkoxy, Nitro oder Cyano bedeuten; und $X^-$ ein einwertiger Säurerest oder der Äquivalent eines mehrwertigen Säurerests ist;

die Komponente d) eine oder mehrere quartäre Phosphoniumverbindungen der Formel (6)

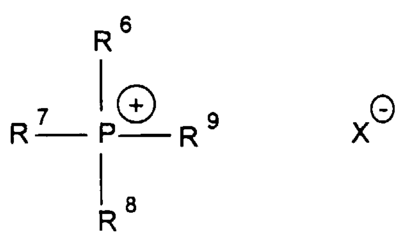

(6)

bedeutet, worin

$R^6$, $R^7$, $R^8$ und $R^9$ gleich oder verschieden sind und
für einen linearen oder verzweigten Alkylrest mit 1 bis 22 Kohlenstoffatomen; oder für einen unsubstituierten oder substituierten Arylrest oder für einen $C_1$-$C_4$-Alkyl-arylrest stehen, wobei Aryl die Bedeutung Phenyl oder Naphthyl hat und die besagten Substituenten Halogen, $C_1$-$C_4$-Alkyl, $C_1$-$C_4$-Alkoxy, Nitro oder Cyano bedeuten; und $X^-$ ein einwertiger Säurerest oder das Äquivalent eines mehrwertigen Säurerests ist;

die Komponente e) einen oder mehrere Polyether der Formel (7)

$$R^{10}\text{-}(O\text{-}C_aH_{2a})_b\text{-}OR^{11} \qquad (7)$$

oder Kronenether bedeutet, wobei in Formel (7)

| | |
|---|---|
| $R^{10}$ und $R^{11}$ | gleich oder verschieden sind und einen geradkettigen oder verzweigten Alkylrest mit 1 bis 16 Kohlenstoffatomen bedeuten; |
| a | eine ganze Zahl von 2 bis 6 und |
| b | eine ganze Zahl von 0 bis 20 ist, |

daß man die Umsetzung bei 50 bis 250°C durchführt, während der Umsetzung die gebildete Verbindung der Formel (1) destillativ abtrennt und die Verbindung der Formel (2), entsprechend wie die Verbindung der Formel (1) abgetrennt wird, der Reaktionsmischung zuführt.

**2.** Verfahren nach Anspruch 1, dadurch gekennzeichnet, daß man eine Verbindung der Formel (2), worin Az unabhängig voneinander gleich oder verschieden ist und für einen Rest -F, -Cl, $-NO_2$, -CN, $-CF_3$, $-CCl_3$, -CHO oder $-CO(C_nH_{2n+1})$ steht, wobei n eine ganze Zahl von 1 bis 6 ist, einsetzt.

**3.** Verfahren nach Anspruch 1 oder 2, dadurch gekennzeichnet, daß man eine Verbindung der Formel (2), worin Ar für einen Phenyl-Rest oder Pyridyl-Rest steht, einsetzt.

**4.** Verfahren nach einem oder mehreren der Ansprüche 1 bis 3, dadurch gekennzeichnet, daß man eine Verbindung der Formel (2), worin y eine ganze Zahl von 1 bis 3 ist, einsetzt.

**5.** Verfahren nach einem oder mehreren der Ansprüche 1 bis 4, dadurch gekennzeichnet, daß man eine Verbindung der Formel (2), worin R für H, einen geradkettigen oder verzweigten Alkylrest mit 1 bis 4 Kohlenstoffatomen oder einen geradkettigen oder verzweigten Alkoxyrest mit 1 bis 4 Kohlenstoffatomen steht, einsetzt.

**6.** Verfahren nach einem oder mehreren der Ansprüche 1 bis 5, dadurch gekennzeichnet, daß man als Alkalimetallfluorid NaF, KF, RbF, CsF oder ein Gemisch derselben einsetzt.

**7.** Verfahren nach einem oder mehreren der Ansprüche 1 bis 6, dadurch gekennzeichnet, daß man die Komponente a) oder die Mischung der Komponente a) mit wenigstens einer der Komponenten b), c), d) und/oder e) in einer Menge von 1 bis 40, insbesondere 2 bis 30, bevorzugt 5 bis 20 Gew.-%, bezogen auf Verbindung der Formel (2) einsetzt.

**8.** Verfahren nach einem oder mehreren der Ansprüche 1 bis 7, dadurch gekennzeichnet, daß man die Umsetzung in Gegenwart der Komponente a) oder der Mischung von Komponente a) und wenigstens einer der Komponenten b) und d) durchführt.

**9.** Verfahren nach einem oder mehreren der Ansprüche 1 bis 8, dadurch gekennzeichnet, daß man als Komponente a) eine oder mehrere quartäre Ammoniumverbindungen der Formel (3), worin

| | |
|---|---|
| $R^1$, $R^2$ und $R^3$ | gleich oder verschieden sind und einen geradkettigen oder verzweigten Rest der Formel $-(C_mH_{2m}O)_pR^5$ bedeuten, worin $R^5$ für Wasserstoff oder einen geradkettigen oder verzweigten Alkylrest mit 1 bis 8 Kohlenstoffatomen steht, m eine ganze Zahl von 1 bis 5 und p eine ganze Zahl von 2 bis 10 bedeuten; oder einen geradkettigen oder verzweigten Alkylrest mit 1 bis 18 Kohlenstoffatomen; oder einen unsubstituierten Phenyl-oder Napththylrest, wobei die Substituenten die Bedeutung Halogen, $C_1$-$C_4$-Alkyl, $C_1$-$C_4$-Alkoxy, Nitro oder Cyano haben; |
| $R^4$ | einen geradkettigen oder verzweigten Alkoxypolyoxyalkyl-Rest der Formel $-(C_mH_{2m}O)_pR^5$ bedeutet, worin $R^5$ für Wasserstoff oder einen geradkettigen oder verzweigten Alkylrest mit 1 bis 8 Kohlenstoffatomen steht, m eine ganze Zahl von 1 bis 5 und p eine ganze Zahl von 2 bis 10 bedeuten; und |
| $X^{\ominus}$ | Fluorid, Chlorid, Bromid, $SO_4^{2-}/2$ oder Hydrogensulfat ist, einsetzt. |

**10.** Verfahren nach einem oder mehreren der Ansprüche 1 bis 9, dadurch gekennzeichnet, daß man Dimethyl-di-(ethoxypolyoxypropyl)-ammoniumchlorid, Dimethyl-di-(ethoxypolyoxypropylmethylether)-ammoniumchlorid, Dimethyl-(ethoxypolyoxypropyl)-(ethoxypolyoxypropylmethylether)-ammoniumchlorid, Dimethyl-di-(ethoxypolyoxyethyl)-ammoniumchlorid, Dimethyl-di-(ethoxypolyoxyethylmethylether)-ammoniumchlorid, Dimethyl-(ethoxypolyoxyethyl)-(ethoxypolyoxyethylmethylether)-ammoniumchlorid, jeweils mit einer mittleren Kettenlänge p von 3, oder Trimethyl-(ethoxypolyoxypropyl)-ammoniumchlorid oder Trimethyl-(ethoxypolyoxypropylmethylether)-ammoniumchlorid, jeweils mit einer mittleren Kettenlänge p von 8, oder ein Gemisch der vorstehend genannten Verbindungen als Komponente a) einsetzt.

**11.** Verfahren nach einem oder mehreren der Ansprüche 1 bis 10, dadurch gekennzeichnet, daß man als Komponente b) ein oder mehrere Amidophosphoniumsalze der Formel (4)

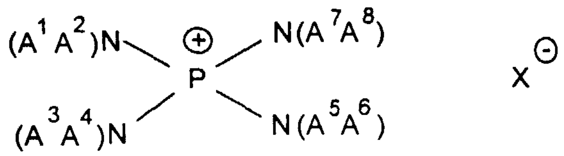

(4)

worin drei Reste $(A^1A^2)N$, $(A^3A^4)N$ und $(A^5A^6)N$ gleich sind und für einen Dialkylaminorest mit 1 bis 6, insbesondere 1 bis 4 Kohlenstoffatomen je Alkyl oder für einen Pyrrolidin-, Piperidin- oder Morpholin-Ring stehen, der Rest $(A^7A^8)N$ sich von den vorstehend genannten Resten unterscheidet, wobei $A^7$ und $A^8$ gleich oder verschieden sind und für einen Alkylrest mit 1 bis 8 Kohlenstoffatomen oder Alkenylrest mit 1 bis 4 Kohlenstoffatomen stehen, oder alle vier Reste $(A^1A^2)N$, $(A^3A^4)N$, $(A^5A^6)N$ und $(A^7A^8)N$ gleich sind und für einen Dialkylaminorest mit 1 bis 6, insbesondere 1 bis 4 Kohlenstoffatomen je Alkyl oder für einen Pyrrolidin-, Piperidin- oder Morpholin-Ring stehen, einsetzt.

**12.** Verfahren nach einem oder mehreren der Ansprüche 1 bis 11, dadurch gekennzeichnet, daß man als Komponente c) ein oder mehrere quartäre Ammoniumverbindungen der Formel (5), worin $R^6$, $R^7$ und $R^8$ gleich sind und für einen Alkylrest mit 1 bis 4, insbesondere 1 bis 2 Kohlenstoffatomen stehen und $R^9$ ein Alkylrest mit 6 bis 24, insbesondere 8 bis 20, bevorzugt 10 bis 18 Kohlenstoffatomen ist, oder $R^6$, $R^7$, $R^8$ und $R^9$ gleich sind und einen Alkylrest mit 1 bis 6, insbesondere 1 bis 4 Kohlenstoffatomen bedeuten, einsetzt.

**13.** Verfahren nach einem oder mehreren der Ansprüche 1 bis 12, dadurch gekennzeichnet, daß man als Komponente d) ein oder mehrere quartäre Phosphoniumverbindungen der Formel (6), worin $R^6$, $R^7$ und $R^8$ gleich sind und für einen Alkylrest mit 1 bis 4, insbesondere 1 bis 2 Kohlenstoffatomen stehen und $R^9$ ein Alkylrest mit 6 bis 24, insbesondere 8 bis 20, bevorzugt 10 bis 18 Kohlenstoffatomen ist, oder $R^6$, $R^7$, $R^8$ und $R^9$ gleich sind und einen Phenylrest oder Alkylrest mit 1 bis 6, insbesondere 1 bis 4 Kohlenstoffatomen bedeuten, einsetzt.

**14.** Verfahren nach einem oder mehreren der Ansprüche 1 bis 13, dadurch gekennzeichnet, daß man als Komponente e) einen oder mehrere Polyether der Formel (7), worin $R^{10}$ und $R^{11}$ gleich oder verschieden sind und einem geradkettigen oder verzweigten Alkylrest mit 1 bis 8 Kohlenstoffatomen bedeuten, a eine ganze Zahl von 2 bis 3 und b eine ganze Zahl von 4 bis 14 ist, oder einen Kronenether einsetzt.